# EUROPÄISCHE PATENTANMELDUNG

(11) **EP 2 266 640 A1**
(43) Veröffentlichungstag der Anmeldung: **29.12.2010**
(21) Anmeldenummer: 09075276.7
(22) Anmeldetag: 25.06.2009
(51) Int. Cl.: A61M 1/10, F04D 3/00, F04D 29/18, F04D 29/24

(54) **Komprimierbares und expandierbares Schaufelblatt für eine Fluidpumpe**

(71) Anmelder: ECP Entwicklungsgesellschaft mbH, 12247 Berlin (DE)
(72) Erfinder: Töllner, Thomas, 12047 Berlin (DE)
(74) Vertreter: Pfenning, Meinig & Partner GbR

(57) **Zusammenfassung**

Die Erfindung bezieht sich auf ein komprimierbares und expandierbares Schaufelblatt (2) für den Rotor einer Fluidpumpe (30) mit wenigstens zwei Lamellen (3,4,5), die nebeneinander angeordnet jeweils gegenüber einer Drehachse (1a) des Rotors schwenkbar und gegeneinander beweglich sind und in expandiertem Zustand derart aneinander anliegen, dass sie gemeinsam eine durchgehende Schaufelblattfläche bilden.

## Beschreibung

Die vorliegende Erfindung liegt auf dem Gebiet der Mechanik bzw. Mikromechanik und kann insbesondere in der medizinischen Technik mit Vorteil angewendet werden.

Die Erfindung bezieht sich auf ein Schaufelblatt für eine Pumpe und dessen Gestaltung. Dabei handelt es sich um eine Pumpe mit einem Rotor, wobei der Rotor komprimierbar und expandierbar ist, um die Gesamtabmaße der Pumpe gegebenenfalls verändern zu können. Auf diese Weise kann die Pumpe durch schwierig zugängliche Öffnungen bzw. in enge Röhrensysteme geschoben werden, wozu sie zunächst komprimiert und, nachdem sie an den Einsatzort gebracht ist, wieder expandiert wird.

Besonders vorteilhaft ist eine solche Pumpe in der Medizintechnik auf dem Gebiet der Herzpumpen oder anderer Pumpen für Körperflüssigkeiten einsetzbar, die üblicherweise mit Kathetern verwendet werden.

Es sind auf dem medizintechnischen Gebiet verschiedene Arten von Mikropumpen bekannt, die in komprimiertem Zustand mit einem Katheter durch ein körpereigenes Gefäß in einen Patientenkörper eingebracht und an Ort und Stelle expandiert werden können. Um eine entsprechende radiale Komprimierbarkeit und Expandierbarkeit zu erzeugen, können verschiedene Effekte bei der Konstruktion und dem Bau des Pumpengehäuses und des Pumpenrotors verwendet werden, wie z. B. der Einsatz von sogenannten Gedächtnislegierungen, die in Abhängigkeit von der Umgebungstemperatur ihre Form ändern, oder durch das Vorsehen bestimmter Übertragungsmechanismen, die es erlauben, den Pumpendurchmesser gezielt zu steuern.

Aus der DE 10 2004 054 714 A1 ist eine Lösung bekannt, bei der sowohl das Laufrad einer Mikropumpe als auch deren Gehäuse durch eine gegenseitige axiale Verschiebung der Pumpenantriebswelle gegenüber einem Katheter manipuliert wird. Dadurch wird das Gehäuse zwischen einem komprimierten und einem expandierten Zustand verändert.

Aus dem Patentdokument WO 00/2003103745 A2 ist ein System bekannt, bei dem das Pumpengehäuse ebenfalls durch eine axiale Relativbewegung zweier Bauteile gegeneinander radial expandiert wird.

Aus der US-Patentschrift US 7393181 ist ein Rotor bekannt, dessen Schaufelblatt zur besseren Komprimierbarkeit/Faltbarkeit in mehrere Teilblätter oder Reihen von Teilblättern unterteilt sein kann. Durch die Unterteilung sind die einzelnen Teilblätter kleiner als eine einteilige Schaufel und nahezu flach, so dass sie leicht einrollbar sind. Allerdings ist die Unterteilbarkeit durch die notwendige zu gewährleistende Eigenstabilität der Teilblätter begrenzt.

Der vorliegenden Erfindung liegt vor dem Hintergrund des Standes der Technik die Aufgabe zugrunde, einen möglichst einfach und kostengünstig herzustellenden Rotor bzw. ein Schaufelblatt für einen solchen Rotor herzustellen, wobei im Vordergrund eine hohe Komprimierbarkeit mit möglichst geringem Kraftaufwand steht, so dass zum Herausziehen der Pumpe aus dem Gefäß die Kompression des Pumpenrotors ohne größere äußere Widerstände realisiert werden kann. Zudem soll die entsprechende Pumpe leistungsfähig und verbrauchsarm gestaltet sein.

Die Aufgabe wird gemäß der Erfindung mit den Merkmalen des Patentanspruchs 1 gelöst.

Die Erfindung sieht ein Schaufelblatt mit mehreren Lamellen vor, die nebeneinander angeordnet, gegeneinander beweglich und gegenüber einer Drehachse des Rotors schwenkbar sind, wobei die Lamellen im expandierten Zustand des Schaufelblattes derart aneinander anliegen, dass sie gemeinsam eine durchgehende Schaufelblattfläche bilden.

Derartige Lamellen lassen sich einzeln durch ihre Schwenkbarkeit sehr leicht in einen Platz sparenden Zustand schwenken und bilden im ausgeschwenkten, expandierten Zustand des Schaufelblatts bzw. des Rotors eine geschlossene Schaufelblattfläche, die derart konzipiert ist, dass sie die notwendigen strömungstechnischen Bedingungen hinsichtlich Formgebung und Oberflächengestaltung erfüllt.

Gegenüber einer faltbaren Membran hat das aus einzelnen Lamellen bestehende Schaufelblatt den Vorteil, dass seine Bestandteile sowohl im komprimierten als auch im expandierten Zustand eine definierte Form und Anordnung einnehmen können. Die einzelnen Lamellen können jeweils für sich an der Welle des Rotors schwenkbar befestigt sein, und zwar vorteilhaft schwenkbar in einer Ebene, die die Längsachse der Rotorwelle enthält oder eine zu einer solchen Welle parallele Achse, wobei die Befestigungspunkte wendelförmig um die Rotorwelle umlaufen können, um im ausgeklappten Zustand ein wendelförmiges Schaufelblatt zu erzeugen. Es sind jedoch auch andere Befestigungsarten denkbar, bei denen die Lamellen beispielsweise an einem oder mehreren Stegen oder Querholmen befestigt sind, die ihrerseits mit der Rotorwelle verbunden sind.

Die einzelnen Lamellen können gleich lang, jedoch auch unterschiedlich lang ausgeführt sein, je nachdem, welche Endform das Schaufelblatt annehmen soll. Die Schwenkbarkeit der einzelnen Lamellen ist begrenzt, so dass diese im aufgeklappten Zustand dem Strömungsgegendruck eines zu befördernden Fluids standhalten können. Die Lamellen können sich jedoch auch gegenseitig dadurch stützen, dass sie aneinander anliegen und gegebenenfalls verriegelnd ineinander greifen. Beispielsweise können die Lamellen nach Art der Elemente einer Vogelfeder konzipiert sein, wobei der Kiel der Feder in dem Vergleich der Rotorwelle entspricht.

Die Konstruktion des Schaufelblattes kann derart konzipiert sein, dass das Schaufelblatt bei Drehung in Betriebsrichtung durch den Fluidgegendruck expandiert und bei Drehung in der entgegengesetzten Richtung durch die Wirkung des Mediums, in dem sich das Schaufelblatt bewegt, komprimiert wird. Dies macht die Kompressions- und Expansionsbewegung besonders einfach, ohne dass größere Kräfte zu überwinden sind. Dadurch lässt sich bei einer Anwendung im medizinischen Gebiet eine derartige Pumpe einfach und ohne eine Gefahr, Körpergefäße zu beschädigen, wieder entfernen.

Eine besondere Ausgestaltung der Erfindung sieht vor, dass wenigstens zwei Lamellen, insbesondere jede der Lamellen für sich formstabil, insbesondere steif ausgebildet ist.

Das Schaufelblatt gewinnt seine Flexibilität nicht durch die Flexibilität einer Membran, sondern durch die Beweglichkeit der einzelnen Lamellen relativ zueinander. Dazu dürfen die Lamellen eine bestimmte Breite nicht überschreiten. Vorteilhaft kann beispielsweise das Schaufelblatt aus mindestens 10 oder mindestens 50 Lamellen bestehen. Ein solches Schaufelblatt kann insgesamt eine sehr kleine Baugröße aufweisen, um in ein Blutgefäß geschoben zu werden, beispielsweise im komprimierten Zustand einen Durchmesser von 2 mm.

Eine weitere vorteilhafte Ausgestaltung der Erfindung kann vorsehen, dass jeweils benachbarte Lamellen entlang einer Längsseite, die sich bezüglich der Rotorachse wenigstens teilweise radial erstreckt, dichtend aneinander anliegen.

Wenn es auch denkbar ist, dass die Lamellen zwischen sich Zwischenräume freilassen, so steigt doch die Effizienz der Fluidpumpe mit der Dichtigkeit des durch die Lamellen gebildeten Schaufelblatts. Es ist daher vorteilhaft, wenn die Lamellen an ihrer Längsseite jeweils aneinanderstoßen und möglichst dort keinen Zwischenraum freilassen.

Es kann zudem vorgesehen sein, dass jeweils einander direkt benachbarte Lamellen derart aneinanderliegen, dass sie um die Rotorachse in wenigstens einem Richtungssinn nicht gegeneinander schwenkbar sind. Damit können sich die einzelnen Lamellen gegenseitig stützen und im Betrieb dem Gegendruck des zu pumpenden Fluids insgesamt standhalten. Insbesondere dann, wenn die Lamellen am Umfang der Rotorwelle wendelförmig verteilt sind, ist eine Stützung in azimutaler Richtung gegeben.

Die Erfindung kann zudem derart ausgestaltet sein, dass jeweils benachbarte Lamellen einander im Bereich der Längsseite überlappen. Durch eine Überlappung der Lamellen wird eine besonders hohe Dichtigkeit erzeugt, und außerdem kann im Überlappungsbereich die Stützfunktion der Lamellen gegenseitig ausgeübt werden.

Eine besonders hohe Stabilität und Dichtigkeit zwischen den Lamellen wird dadurch erreicht, dass jeweils benachbarte Lamellen im Bereich der Längsseite ineinandergreifen. Dabei kann jede Art von formschlüssiger Gestaltung benachbarter Lamellen vorgesehen sein, beispielsweise jeweils das Vorsehen einer Falz entlang der Längsseiten der Lamellen, beispielsweise auch in Form einer dünnen Dichtlippe.

Um eine formschlüssige und dichte Verbindung herzustellen, kann auch vorgesehen sein, dass wenigstens eine Lamelle, insbesondere alle Lamellen, im Querschnitt an einer ihrer Längsseiten eine konvexe Form und an der anderen Längsseite eine konkave Form aufweist. Die entsprechende konvexe oder konkave Struktur kann im Querschnitt rund, elliptisch oder auch als Nut oder Einkerbung gestaltet sein.

Vorteilhaft können jeweils benachbarte Lamellen durch ein biegeschlaffes Element, insbesondere ein Band oder eine Membran, miteinander verbunden sein. Die Ausklappbarkeit des Schaufelblattes ergibt sich dann nach Art eines Fächers, bei dem breite, steife Stützstäbe durch schmale Membranen oder Bänder miteinander verbunden sind.

Zur Stabilisierung der einzelnen Lamellen können diese jeweils im Querschnitt eine Versteifungsstruktur aufweisen, die beispielsweise einen in Längsrichtung verlaufenden Steg vorsieht. Es kann jedoch auch vorgesehen sein, dass zusätzlich oder alternativ die einzelnen Lamellen hohl mit rundem oder eckigem Querschnitt ausgebildet sind.

Vorteilhaft kann die Erfindung ausgebildet werden durch ein Schaufelblatt bei dem wenigstens zwei Lamellen durch eine klettartige Verbindung miteinander verbunden sind. Eine solche klettartige Verbindung besteht aus kleinen hakenartigen Elementen auf der einen und Schlaufenartigen Elementen auf der jeweils anderen Seite, die vorteilhaft mikroskopisch klein ausgebildet sein können.

Die Erfindung kann weiter dadurch vorteilhaft ausgestaltet sein, dass die Verbindung durch Aufbringen einer Belastung auf das Schaufelblatt in Axialrichtung des Rotors und/oder durch Relativbewegung zweier benachbarter Lamellen entlang ihrer jeweiligen Längsseiten und in Längsrichtung der Lamellen lösbar ist.

Die Längsrichtung der Lamellen ist dabei durch die Richtung gegeben, in der sich die jeweilige Lamelle von der Rotorwelle weg erstreckt.

Durch die beschriebene Struktur eines Schaufelblattes für den Rotor einer Fluidpumpe kann dieses besonders stabil und definiert komprimierbar gestaltet werden, wodurch sich insbesondere eine gute Komprimierbarkeit und ein geringer Enddurchmesser des Rotors in der komprimierten Form erreichen lässt.

Die Erfindung wird im Folgenden anhand eines Ausführungsbeispiels in einer Zeichnung gezeigt und anschließend beschrieben.

Dabei zeigt:
- Fig. 1: schematisch in einer dreidimensionalen Ansicht eine Rotorwelle sowie ein Schaufel- blatt,
- Fig. 2: schematisch einen Teil eines Schaufel- blattes mit mehreren Lamellen,
- Fig. 3: eine Rotorwelle im Querschnitt mit einer Lamelle in zwei Positionen,
- Fig. 4: eine Rotorwelle im Querschnitt mit zwei Lamellen in jeweils zwei Positionen,
- Fig. 5: eine Ansicht einer Rotorwelle mit vier Lamellen,
- Fig. 6: eine Ansicht einer Rotorwelle mit zwei Konfigurationen von Lamellen,
- Fig. 7: eine Ansicht einer Rotorwelle mit zwei Konfigurationen von Lamellen sowie
- Fig. 8: eine schematische Darstellung einer Herz- katheterpumpe mit einem Rotor und Schaufel- blättern in einer Herzkammer,
- Figuren 9 bis 12: zeigenschematische 3-dimensionale Darstellungen überlappender Lamellen.

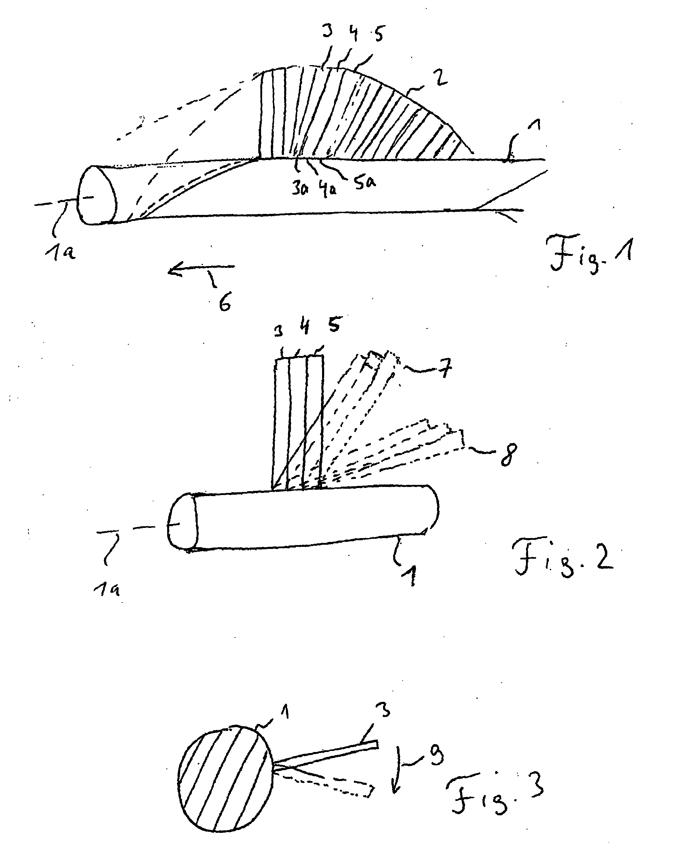

Fig. 1 zeigt eine Rotorwelle 1 mit einem Schaufelblatt 2, das aus einzelnen, schematisch angedeuteten Lamellen 3, 4, 5 zusammengesetzt ist. Die einzelnen Lamellen sind jeweils mit ihren Fußpunkten 3a, 4a, 5a auf der Rotorwelle 1 schwenkbar befestigt, wobei die Fußpunkte der Lamellen insgesamt wendelförmig um die Rotorwelle 1 umlaufen.

Auf diese Weise wird eine helixförmige Struktur eines Schaufelblattes geschaffen, die bei Rotation um die Rotorwelle 1 eine axiale Beförderung einer Flüssigkeit in Richtung des Pfeils 6 bewirkt.

Die besondere Ausgestaltung des erfindungsgemäßen Schaufelblattes geht detaillierter aus der Fig. 2 hervor. Dort sind in einer ersten Position die Lamellen 3, 4, 5 in aufgespannter, expandierter Form des Schaufelblattes dargestellt, wobei die benachbarten Lamellen mit ihren Längsseiten eng aneinander anliegen und somit eine für das strömende Fluid glatte und dichte Oberfläche bilden.

In der Position, die gestrichelt dargestellt und mit 7 bezeichnet ist, sind die einzelnen Lamellen ein Stück weit an die Rotorwelle 1 angeklappt, wobei für die Deformierbarkeit des Schaufelblattes insgesamt wichtig ist, dass die einzelnen Lamellen 3, 4, 5 gegeneinander beweglich, insbesondere in Längsrichtung verschiebbar sind. Dadurch ist kein Falten der entsprechenden Fläche notwendig, sondern die einzelnen Lamellen können bis weit zur Rotorwelle hin angeklappt werden, wie in der weiteren Position 8 der Lamellen dargestellt ist.

Hierdurch lässt sich das Schaufelrad weitgehend komprimieren, d. h. bezüglich des Radius, bezogen auf die Rotorwelle 1 oder deren Längsachse 1a reduzieren.

Es entstehen dabei auch keine nennenswerten elastischen Gegenkräfte, so dass der Rotor praktisch kraftfrei komprimiert werden kann, wenn dies beispielsweise zum Einführen oder Entfernen einer entsprechenden Fluidpumpe aus einem körpereigenen Gefäß notwendig ist.

Ist in der Fig. 2 eine Schwenkbarkeit der einzelnen Lamellen in Längsrichtung der Rotorwelle 1 in der Ebene der Rotorwellenachse angedeutet, so ist die Erfindung hierauf jedoch nicht beschränkt. Fig. 3 zeigt eine Schwenkbarkeit einer Lamelle 3 in azimutaler Richtung, wie durch den Pfeil 9 angedeutet.

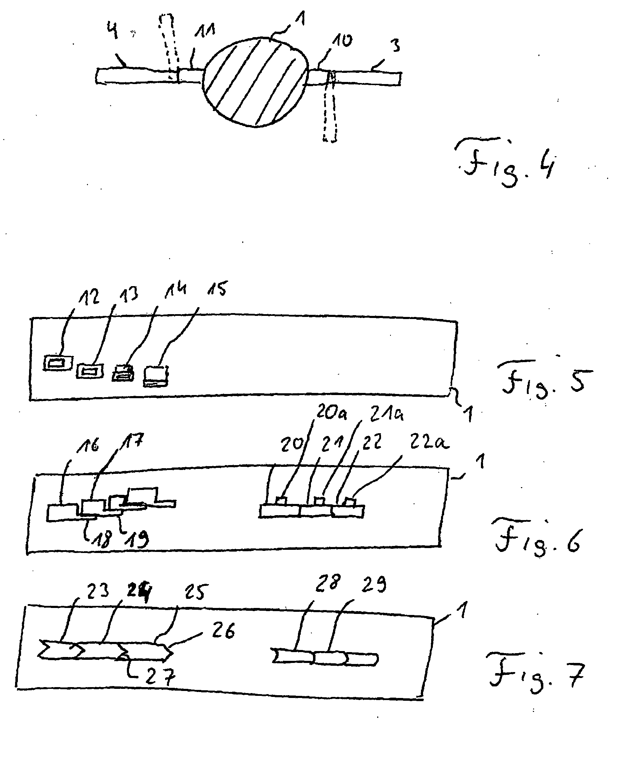

Fig. 4 zeigt eine weitere Variante einer solchen Ausgestaltung, wobei Stege 10, 11 an der Rotorwelle 1 vorgesehen sind und die Lamellen 3, 4 auf den wendelförmig um die Rotorwelle 1 umlaufenden Stegen 10, 11 schwenkbar befestigt sind. Die geschwenkten Positionen sind in der Fig. 4 jeweils gestrichelt dargestellt.

Es wird deutlich, dass das Schwenken der Lamellen in der jeweils gestrichelt dargestellten Position zu einer Komprimierung des Rotors führt. Beispielsweise kann eine Komprimierung des Rotors durch einen Drehbetrieb des Rotors in einer der Betriebsrichtung entgegengesetzten Richtung hervorgerufen werden. Das Aufspannen des Rotors geschieht entsprechend durch Rotation in der Betriebsrichtung.

Grundsätzlich können die einzelnen Lamellen auch an Querholmen der Rotorwelle 1 befestigt sein und im aufgespannten Zustand parallel zur Längsachse der Rotorwelle verlaufen. Wichtig ist, dass sie zur Durchmesserreduktion des Rotors entsprechend einzeln einklappbar sind.

In der Fig. 5 ist schematisch eine Draufsicht auf vier Lamellen 12, 13, 14, 15 gezeigt, die jeweils im Querschnitt rechteckig und hohl ausgebildet sind, um eine größere Längssteifigkeit der einzelnen Lamellen zu erzeugen. Zielführend ist dabei, dass trotz der Steifigkeit der einzelnen Lamellen das gesamte Schaufelblatt einfach einklappbar ist.

Die Fig. 6 zeigt zwei Konfigurationen von Lamellen, wobei auf der linken Seite jeweils Lamellen 16, 17 dargestellt sind, die eine Überdeckungslippe 18, 19 aufweisen, wobei benachbarte Lamellen jeweils an der Überdeckungslippe 18, 19 der Nachbarlamelle einerseits dichten und andererseits abgestützt sind. Dadurch ergibt sich eine Versteifung des gesamten Schaufelblattes, so dass das Schaufelblatt einem erhöhten Fluidgegendruck im Betrieb standhält.

Auf der rechten Seite der Fig. 6 sind drei Lamellen 20, 21, 22 dargestellt, wobei jede der Lamellen einen in Radialrichtung der Rotorwelle 1 verlaufenden Steg 20a, 21a, 22a aufweist.

Fig. 7 zeigt auf der linken Seite der Draufsicht auf die Rotorwelle 1 drei Lamellen 23, 24, 25, die jeweils an ihrer Längsseitenfläche auf einer Seite 26 eine konvexe Ausstülpung, auf der anderen Seite 27 eine konkave Einbuchtung aufweisen, damit benachbarte Lamellen ineinandergreifen und sich so gegenüber einer azimutalen Schwenkbewegung gegenseitig stützen können.

Auf der rechten Seite der Fig. 7 sind Lamellen 28, 29 dargestellt, wobei jede der Lamellen auf ihren Längsseiten eine konkave und eine konvexe Ausbuchtung mit rundem Querschnitt aufweisen. Diese Gestaltung hat den Vorteil, dass benachbarte Lamellen gegeneinander beschränkt um ihre Längsachse drehbar sind.

Grundsätzlich können die einzelnen Lamellen entweder mittels eines Schwenkgelenkes an der Rotorwelle 1 befestigt oder in ihrem Fußbereich derart biegsam bzw. flexibel ausgestaltet sein, dass sie jedenfalls als Ganzes gegenüber der Rotorwelle schwenkbar sind. Die einzelnen Lamellen können auch mit ihren Fußenden jeweils einzeln auf ein flexibles Band aufgeklebt oder an diesem auf andere Weise befestigt sein, wobei das Band als Ganzes mit den Lamellen auf dem Rotor befestigt werden kann. Durch die Flexibilität des Bandes kann dann die Schwenkbarkeit der einzelnen Lamellen gewährleistet sein.

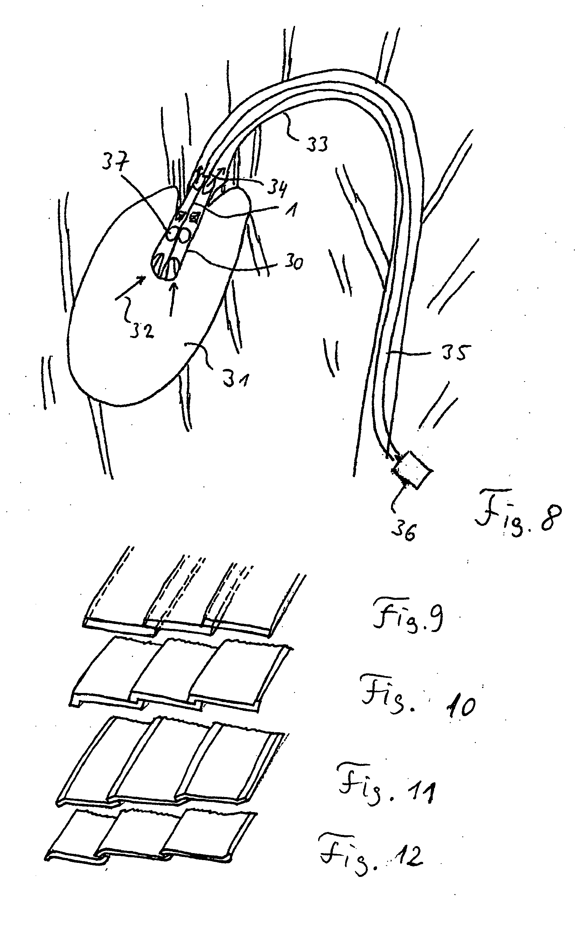

In der Fig. 8 ist schematisch der Einsatz einer Fluidpumpe mit einem erfindungsgemäßen Schaufelblatt dargestellt, wobei die Pumpe 30 in einer Herzkammer 31 positioniert ist und, wie durch die Pfeile 32 angedeutet, Blut ansaugt, das in ein Gefäß 33 befördert wird, wie dies durch die Pfeile 34 gezeigt ist. Die Pumpe 30 ist auf einem Katheter 35 befestigt, durch den zentral eine nur im Bereich der Pumpe 30 dargestellte Welle 1 verläuft, die mittels eines Motors 36 rotatorisch angetrieben wird. Die Welle bewegt einen Rotor 37, der ein nur schematisch dargestelltes Schaufelblatt aufweist.

Die Pumpe 30 weist im expandierten Zustand einen Durchmesser auf, der im Extremfall möglicherweise auch größer als der Innendurchmesser des Gefäßes 33 sein kann. Zu diesem Zweck wird das Schaufelrad voll expandiert. Es kann jedoch zum Einbringen oder Entnehmen der Pumpe 30 auch komprimiert werden, wobei die einzelnen Lamellen, wie oben dargestellt, an die Rotorwelle 1 angeklappt werden können und gleichzeitig das Gehäuse der Pumpe 30 entsprechend kollabiert. Dazu kann dieses Gehäuse beispielsweise aus einer Membran bestehen, die durch ein Gerüst oder durch den in der Pumpe 30 erzeugten Fluiddruck aufgespannt wird.

Figur 9 zeigt drei flache, an ihren Längsseiten überlappende Lamellen, die z.B. in ihrem Überlappungsbereich eine Klettverbindung aufweisen können.

Figur 10 zeigt Lamellen, die entlang ihrer Längsseiten jeweils um 90 Grad abgewinkelte Kanten aufweisen, mit denen sie ineinander einhaken, während in Figur 11 eine Variante mit einer Abwinkelung um weniger als 90 Grad dargestellt ist, welche ebenfalls eine Fixierung der Lamellen relativ zueinander erlaubt.

Figur 12 schließlich stellt eine Variante mit einer geschwungenen Kante dar, die demselben Zweck der gegenseitigen Fixierung dient.

## Patentansprüche

1. Komprimierbares und expandierbares Schaufelblatt (2) für den Rotor einer Fluidpumpe, **gekennzeichnet durch** wenigstens zwei Lamellen (3,4,5,12,13,14,15,16,17,20,21,22,23,24,25,28, 29), die nebeneinander angeordnet jeweils gegenüber einer Drehachse (1a) des Rotors schwenkbar und gegeneinander beweglich sind und in expandiertem Zustand derart aneinander anliegen, dass sie gemeinsam eine durchgehende Schaufelblattfläche bilden.

2. Schaufelblatt nach Anspruch 1, **dadurch gekennzeichnet, dass** wenigstens zwei Lamellen (3,4, 5,12,13,14,15,16,17,20,21,22,23,24,25,28,29), insbesondere jede der Lamellen für sich formstabil, insbesondere steif ausgebildet sind.

3. Schaufelblatt nach Anspruch 1 oder 2, **dadurch gekennzeichnet, dass** jeweils benachbarte Lamellen (3,4,5,12,13,14,15,16,17,20,21,22,23,24, 25,28,29), entlang einer Längsseite, die sich bezüglich der Rotorachse (1a) wenigstens teilweise radial erstreckt, dichtend aneinander anliegen.

4. Schaufelblatt nach Anspruch 2 oder 3, **dadurch gekennzeichnet, dass** jeweils einander direkt benachbarte Lamellen (16,17,20,21,22,23,24,25,28, 29), derart aneinanderliegen, dass sie um die Rotorachse (1a) in wenigstens einem Richtungssinn nicht gegeneinander schwenkbar sind.

5. Schaufelblatt nach Anspruch 3 oder 4, **dadurch gekennzeichnet, dass** jeweils benachbarte Lamellen (16,17) einander im Bereich der Längsseite überlappen.

6. Schaufelblatt nach Anspruch 3 oder 4, **dadurch gekennzeichnet, dass** jeweils benachbarte Lamellen (23,24,25,28,29) im Bereich der Längsseite ineinandergreifen.

7. Schaufelblatt nach Anspruch 1 oder einem der folgenden, **dadurch gekennzeichnet, dass** jeweils benachbarte Lamellen (3,4,5,12,13,14,15,16,17, 20,21,22,23,24,25,28,29), durch ein biegeschlaffes Element, insbesondere ein Band oder eine Membran, miteinander verbunden sind.

8. Schaufelblatt nach Anspruch 1 oder einem der folgenden, **dadurch gekennzeichnet, dass** wenigstens eine der Lamellen (20,21,22) im Querschnitt eine Versteifungsstruktur (20a,21a,22a) aufweist.

9. Schaufelblatt nach Anspruch 8, **dadurch gekennzeichnet, dass** wenigstens eine Lamelle(20,21,22) auf ihrer Außenseite wenigstens einen in ihrer Längsrichtung verlaufenden Versteifungssteg (20a,21a,22a) aufweist.

10. Schaufelblatt nach Anspruch 8, **dadurch gekennzeichnet, dass** wenigstens eine Lamelle (12,13, 14,15) im Querschnitt hohl ausgebildet ist.

11. Schaufelblatt nach Anspruch 8, 9 oder 10, **dadurch gekennzeichnet, dass** wenigstens eine Lamelle (28,29) im Querschnitt an einer ihrer Längsseiten eine konvexe Form und an der anderen Längsseite eine konkave Form aufweist.

12. Schaufelblatt nach Anspruch 1 oder einem der folgenden, **dadurch gekennzeichnet, dass** wenigstens zwei Lamellen (3,4,5,12,13,14,15,16,17, 20,21,22,23,24,25,28,29) durch eine klettartige Verbindung miteinander verbunden sind.

13. Schaufelblatt nach Anspruch 12, **dadurch gekennzeichnet, dass** die Verbindung durch Aufbringen einer Belastung auf das Schaufelblatt in Axialrichtung des Rotors lösbar ist.

14. Schaufelblatt nach Anspruch 12, **dadurch gekennzeichnet, dass** die Verbindung durch Relativbewegung zweier benachbarter Lamellen (3,4,5,12,13, 14,15,16,17,20,21,22,23,24,25,28,29) entlang ihrer jeweiligen Längsseiten und in Längsrichtung der Lamellen lösbar ist.
